# EUROPEAN PATENT APPLICATION

(11) **EP 1 359 214 A1**
(43) Date of publication of application: **05.11.2003**
(21) Application number: 02076795.0
(22) Date of filing: 03.05.2002
(51) Int. Cl.: C12M 3/00

(54) **Bioreactor**

(71) Applicant: IsoTis N.V., 3723 MB Bilthoven (NL)
(72) Inventor: Oostra, Jaap, 3768 EJ Soest (NL)
(74) Representative: Prins, Adrianus Willem

(57) **Abstract**

Bioreactor, comprising a rigid, portable outer housing enclosing a sterile medium chamber in which an inner housing is disposed. The outer housing is provided with a medium inlet port and a medium outlet port for sustaining a medium flow path through the medium chamber and with a medium chamber opening. The medium chamber opening is openable and closable with an outer housing lid for allowing placement and removal of the inner housing. The inner housing is disposed in the flow path and has a medium permeable wall enclosing a growth chamber for growing cells on three-dimensional biomaterial scaffolds to a hybrid tissue engineered construct. The inner housing further comprises a growth chamber opening, closable with an inner housing lid for allowing placement of the scaffolds and removal of hybrid constructs grown therewith. The inner housing further comprises a sterile gripping surface, accessible when said medium chamber is open, for sterile removal of the inner housing from the outer housing.

## Description

The invention relates to a bioreactor.

Bioreactors are generally known and can be used to engineer human tissues ex vivo for transplantation. A major drawback with the known bioreactors is recovery and transportation of three-dimensional tissues from a production facility to a medical operation chamber without incurring chance of contamination.

The invention aims to provide a bioreactor that can receive, maintain and grow cells on a three dimensional biomaterial scaffold to a hybrid tissue construct that can be transported to an operation chamber, be removed from the bioreactor and made available without exposure of the sterile tissue to the external environment during transport.

According to the invention this problem is solved by providing a bioreactor, comprising a rigid, portable outer housing enclosing a sterile medium chamber in which an inner housing is disposed, the outer housing being provided with a medium inlet port and a medium outlet port for sustaining a medium flow path through the medium chamber and with a medium chamber opening, openable and closable with an outer housing lid for allowing placement and removal of the inner housing, said inner housing being disposed in said flow path and having a medium permeable wall enclosing a growth chamber for growing cells on three-dimensional biomaterial scaffolds to a hybrid tissue engineered construct, a growth chamber opening, openable and closable with an inner housing lid for allowing placement of the scaffolds and removal of the hybrid constructs grown therewith, and a sterile gripping surface, accessible when said medium chamber is open, for sterile removal of the inner housing from the outer housing. This way, patient specific tissue can be grown and transported to the operation chamber and made available in sterile condition when it is needed in a simple and elegant manner, with a minimum chance of contamination.

Further advantageous embodiments of the invention are described in the dependent claims.

The invention shall be elucidated further using two exemplary embodiments shown in a drawing. The drawing shows:
Fig. 1A a schematical cross-section of a first embodiment of the outer housing of a first embodiment of the bioreactor;
Fig. 1B a schematical cross-section of the inner housing of the bioreactor of fig. 1A;
Fig. 1C and 1D, schematical perspective top and bottom views of the inner housing of fig. 1B;
Fig. 2A schematic process scheme in which the bioreactor of fig. 1 is included;
Fig. 3A-D schematical perspective views of a second embodiment of the bioreactor in subsequent steps of dismantling; and
Fig. 4 a cross-section of the bioreactor of fig. 3.

It should be noted that the figures are schematical representations of exemplary embodiments of the invention, which are given as non-limitative examples. In the drawings, corresponding parts of the first and second embodiments are provided with the same reference numerals, the reference numerals of the second embodiment being preceded by the prefix 1.

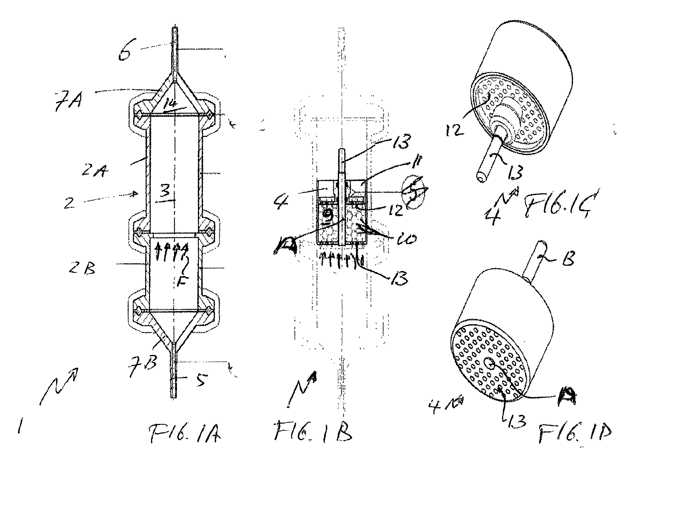

Fig. 1 shows a first embodiment of a bioreactor of the perfusion type. The bioreactor 1 comprises a rigid, portable outer housing 2 enclosing a sterile medium chamber 3 in which an inner housing 4 is disposed. The outer housing 2 is provided with a medium inlet port 5 and with a medium outlet port 6 for sustaining an axial medium flow path F through the medium chamber 3. The outer housing 2 has an outer housing opening 14 openable and closable with an outer housing lid 7 which allows for placement and removal of the inner housing 4. The inner housing 4 is disposed in the flow path F and has a medium permeable wall enclosing a growth chamber 9 for three-dimensional biomaterial scaffolds 10. Within this context, the term biomaterial scaffold is to be construed as a scaffold made of biocompatible material that is suitable for growing bio-material such as cells thereon and therein to a hybrid construct of tissue and scaffold material.

The inner housing 4 has a growth chamber opening 11, closable with an inner housing lid 12 for allowing placement and removal of the scaffolds 10. The inner housing 4 further has a sterile gripping surface 13, accessible when the medium chamber 3 is open so that the inner housing 4 can be removed from the outer housing 2 in sterile condition.

The inner housing 4 and the outer housing 2 are configured as coaxially disposed, nested cylinders. In this embodiment, the outer housing 2 has a tubular body that is built-up from two tubes 2A, 2B of different length that are sealingly clamped together. By choosing the length of the tubes 2A, 2B, the reactor volume can be varied. The tubular body is on both ends closed with outer housing lids 7 which are sealingly clamped on the tubular body. The lids 7 and the tubular parts are sealingly connected via press-fit O-rings. The outer housing lids each taper into an aseptic connector forming the inlet port 5 and outlet port 6 respectively.

The inner housing 4 is designed as a rigid basket for the scaffolds 10 and is in this embodiment formed as a substantially cylindrical cup with a central axis A protruding from the inner housing to form the sterile gripping surface 13. The inner housing lid 12 is slidably carried on the central axis A, so that the scaffolds 10 can be press-fit in the growth chamber 9. The inner housing lid 12 and the bottom 13 of the cup-shaped inner housing 4 are permeable to the medium and define the end surfaces of the cylindrical inner housing 4. The inner housing 4 is placed in the flow path F for axial flow.

In operation, medium enters the reactor 1 at the medium inlet port 5 and is forced to flow to the scaffolds 10 that are kept press-fit in the growth chamber 9. Cells are cultivated on the scaffolds and are nourished by the medium passing through the growth chamber 9. After perfusion of the growth chamber 9, the medium is partially depleted of its nutrients and exits the reactor 1 via the outlet port 6.

The bioreactor 1 is assembled as a disposable unit from plastics material, e.g. polycarbonate (PC), polyethylene (PE) or polypropylene (PP). Before operation, the bioreactor 1 is assembled by placing scaffolds 10 in the growth chamber 9 of the inner housing 4 and by closing with the inner housing lid 12. Subsequently, the inner housing 4 is placed in the medium chamber 3 of the outer housing and the outer housing 2 is closed. After assembly the reactor is sterilized, e.g. by γ radiation.

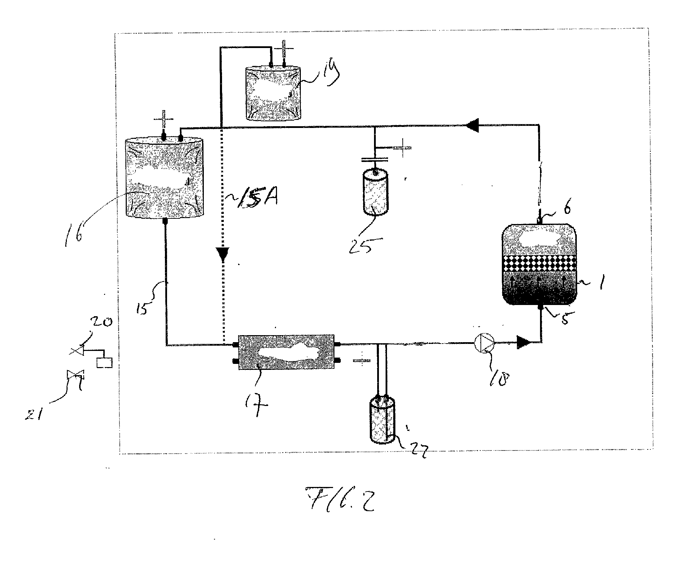

Referring to fig. 2, an exemplary process scheme is shown for growing mesenchymal progenitor cells ex vivo in the bioreactor 1. The bioreactor is included in a medium circulation loop 15 in which medium flows from a medium bag 16 through an oxygenator 17 under the action of a pump 18 through the bioreactor 1. The medium is recirculated through the medium bag 16, and, after depletion of its nutrients, fed to a waste bag 19. The oxyenator 17 is connected to an air supply 20 and to a CO₂ supply 21. It shall be clear that the supplies 21, 21 can be used to provide different gases or gas mixtures.

During start-up, medium is pumped through seeding loop 15A which runs through an inoculation vessel 22. A cell suspension is transported by the medium from the inoculation vessel 22 to the scaffolds 10 in the bioreactor onto which they adhere. After seeding, normal circulation starts and the cells grow on and into the scaffolds 10. During normal circulation, medium is sampled discontinuously by collecting medium in a sampling bag 25. It shall be clear that, as an alternative, medium can flow from the medium bag 16 to the waste bag 19 without recirculation.

Once the cultivation process is completed, the bioreactor 1 is disconnected from the medium circulation loop 15 by unfastening its aseptic connectors at the medium inlet port 5 and the medium outlet port 6. Subsequently, the bioreactor 1 is transported under suitable conditions to the surgical environment. In the sterile environment, a surgical assistant holds the non-sterile outer housing 2 of the bioreactor 1 and removes the outer housing lid 7 and presents the opening 14 of the outer housing 2 to a surgeon who in turn grips the sterile gripping surface 13 on the central axis and removes the inner housing 4 from the outer housing 2 in sterile condition. Under sterile conditions, the inner housing lid 12 is subsequently removed, so that the tissue engineered constructs can be taken out and used in the patient. Finally, the bioreactor 1 is disposed of.

This way, autologous tissue can be grown and transported to the operation chamber and made available in sterile condition when it is needed in a simple and elegant manner, with a minimum chance of contamination.

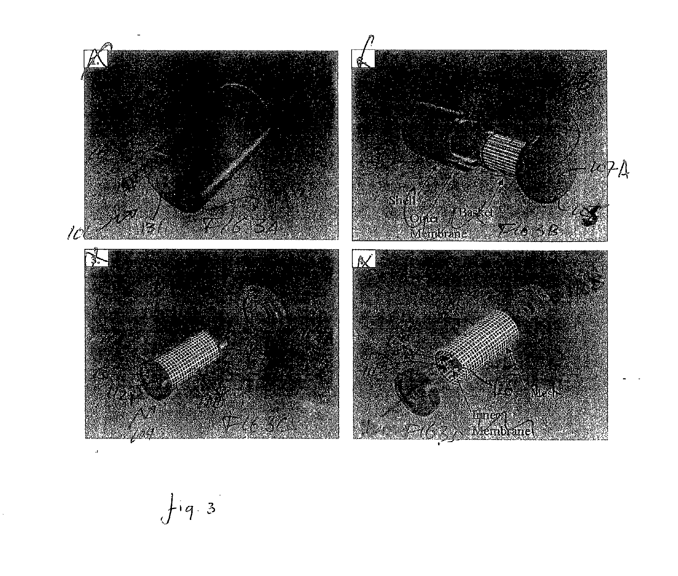

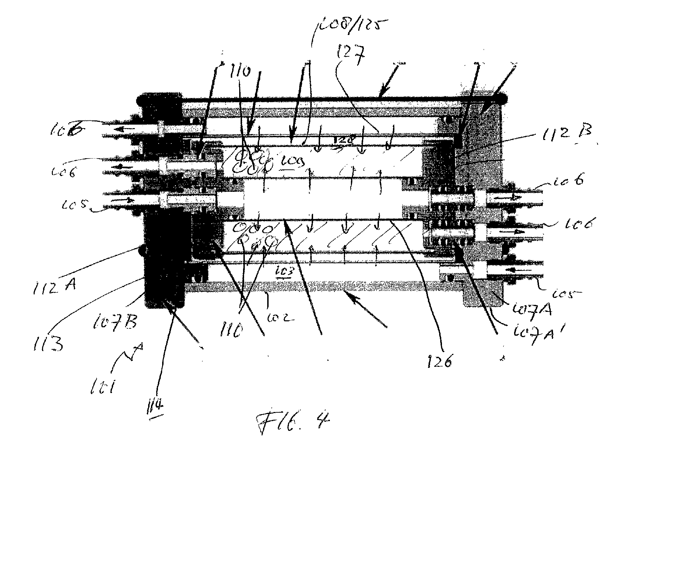

Referring to fig. 3 and 4, an alternative embodiment of the bioreactor 1 based on diffusion or reduced medium flow is shown.

Fig. 3A shows the bioreactor 101 with its outer housing 102 formed as a cylinder. The outer housing 102 is closed with outer housing lids 107A and 107B. In the outer housing lids 107, a medium inlet ports 105 and medium outlet ports 106 are provided.

Referring to fig. 3B it is shown that the bioreactor 101 encloses a medium chamber 103 in which an inner housing 104 is placed.

Referring to fig. 3C, the inner housing 104 is removably carried on the outer housing lid 107. Referring again to fig. 3B, the inner housing 104 can be removed from the medium chamber 103 by gripping on of the rim 107A' of the outer housing lid 107A and by retracting the outer housing lid 107A with the inner housing attached thereto from the medium chamber 103.

The inner housing 104 has a cylindrically shaped medium permeable wall 108 that is disposed in the flow path through the medium chamber 103 extending between the medium inlet port 105 and the medium outlet port 106 and which will be elucidated further. The permeable wall 108 of the inner housing 104, in this embodiment a wire mesh or gauze mantle surface 125 of the cylinder, is interposed between an inner membrane 126 and an outer membrane 127 placed in the medium chamber 103.

The inner and outer housings 102, 104 are configured as nested cylinders. It is observed that the cylinders as shown are circular cylinders, but it shall be clear that for the purpose of nesting, the shape of the inner and outer housing may vary. The mantle surface 125 of the cylindrical inner housing 104 is arranged in the flow path F for radial flow. Referring to fig. 3C, the inner housing 104 comprises a sterile gripping surface 113 disposed on an inner housing lid 112A. The inner housing 104 can be gripped by a person whose hands are free from contamination gripping the sterile gripping surface 113 and pulling the inner housing 104 free from the outer housing lid 107 held by a person at its rim 107A' that may be contaminated. Removal of the inner housing 104 from the outer housing lid 107 can e.g. take place by unscrewing a screw-thread connection, by pulling a press-fit connection loose, of by undoing a bayonet connection.

Referring to fig. 3D, the inner housing 104 has a medium permeable mantle surface 125 formed by the cylindrical permeable wall 108 that is closed off to form a cylinder by two inner housing lids 112A, 112B. The inner housing 104 encloses a growth chamber 109 in which scaffolds 110 are packed between the mantle 125 formed by permeable wall 108 and a tubular inner membrane 126. Preferably, the mantle surface 125 opens to a C-shape under spring action after removal of the inner housing lids 112.

By removal of the inner housing lids 112, the growth chamber opening 111 is opened and the tissue engineered product can be removed. Between the mantle surface 125 and the outer membrane 127 an inoculation chamber 128 is formed in the flow path. The inoculation chamber 128 can optionally be connected to an inoculation port (not shown) in the outer housing 2 located opposite to the outer housing lid 107. Likewise, a construct sampling port (not shown) can be provided extending through both an outer housing lid 107 and an inner housing lid 112 allowing access to the growth chamber 109.

Preferably, a construct sampling chamber is provided in the growth chamber 5 as a removable unit to prevent contamination. Such a removable construct sampling chamber has a medium permeable wall and holds a number of sampling scaffolds and is preferably removed through the construct sampling port via a lock or sluice system forming an aseptic barrier between the growth chamber 109 and the outer housing 102.

Operation of the bioreactor 101 is substantially the same as described above, except for the fact that the seeding loop 15A can be omitted when a seeding chamber 129 and a seeding port are provided to charge the bioreactor with cells. To bring the cells into contact with the scaffolds, the bioreactor 101 can e.g. be rotated or lightly shaken.

Referring to fig. 4, a cross section of the bioreactor101 is shown. The inner membrane 126 and the outer membrane 127 can be a reduced flow membrane or a diffusion membrane depending on the pore size of the membrane. If needed, the membranes 126, 127 can be reinforced or carried on a protective medium permeable wall. Alternatively, the growth chamber 10 can be provided with a spaced permeable wall around the inner membrane, such that the scaffolds are enclosed in an annular space or scaffold chamber between the mantle 125 and the protective wall, which is in turn interposed between the outer 126 and inner 127 membranes spaced thereon.

The way of operating a bioreactor and the type of medium are known to the skilled person. Examples of suitable media and methods for operating the bioreactor can be found in e.g.:
- Botchwey EA, Pollack SR, Levine EM, Laurencin CT. 2001. Bone tissue engineering in a rotating bioreactor using a microcarrier matrix system. J Biomed Mater Res 55: 242-252
- Friedstein AJ, Chailakhyan RK, Gerasimov UV. 1987. Bone marrow osteogenic stem cells: In vitro cultivation and transplantation in diffusion chambers. Cell Tissue Kinet 20: 263-272
- Goldstein A. 2001. Effect of Seeding Osteoprogenitor Cells as Dense Clusters on Cell Growth and Differentiation. Tissue Eng 7: 817-827
- Koller MR, Bradley MS, Palsson B. 1995. Growth factor consumption and production in perfusion cultures of human bone marrow correlate with specific cell production, Experimental Haematology 23:1275-1283
- Mendes SC, Tibbe JM, Veenhof M, Bakker K, Both S, Platenburg PP, Oner FC, de Bruijn JD, van Blitterswijk CA. 2002. Bone tissue engineered implants using bone marrow stromal cells: effect of culture conditions and donor age. Tissue Eng, accepted.
- Strehl R, Schumacher K, De Vries U, Minuth W. 2002. Proliferating cells versus Differentiated Cells in Tissue Engineering. Tissue Eng 8: 37-42.
- Van der Pol JJ, Spohn U, Eberhardt R, Gätgens J, Biselli M, Wandrey C, Tramper J. 1994. On-line monitoring of an animal cell culture with multi-channel Flow injection analysis, Journal of Biotechnology 37: 253-264

Suitable scaffolds are three-dimensional porous macro carriers, e.g. hollow fibre cartridges, porous ceramic scaffolds or polymer scaffolds. Examples of such scaffolds can be found in e.g.:
- Blunk T, Sieminski AL, Gooch KJ, Courter DL, Hollander AP, Nahir AM, Langer R, Vunjak-Novakovic G, Freed LE. 2002. Differential effects of growth factors on tissue-engineered cartilage. Tissue Eng 8:73-84
- Bruder SP, Fox BS. 1999. Tissue engineering of bone. Clin Orthop 367S:68-83
- De Bruijn JD, Van den Brink I, Mendes S, Dekker R, Bovell YP, Van Blitterswijk CA. 1999. Bone induction by implants coated with cultured osteogenetic bone marrow cells. Adv Dental Res 13:74-81
- Goshima J, Goldberg VM, Caplan AI. 1991. The origin of bone formed in composite grafts of porous biphasic calcium phosphate ceramic loaded with marrow cells. Clin Orthop 269: 274-283
- Ishida H, Ohgushi H, Inoue K, Yoshikawa T, Yajima H, Tamai S, Dophi Y. 1996. Cells and tissue induce bone formation in porous hydroxyapatite ceramics. Bioceramics 9:73-76.
- Iyoda K, Miura T, Nogami H. 1993. Repair of bone defect with cultured chondrocytes bound to hydroxyapatite. Clin Orthop 288:287-293
- Kadiyala S, Jaiswal N, Bruder SP. 1997. Culture expanded, bone marrow-derived mesenchymal stem cells can regenerate a critical-sized segmental bone defect. Tissue Eng 173
- Klawitter JJ, Bagwell JG, Weinstein AM, Sauer BW. 1976. An evaluation of bone growth into porous high-density polyethylene. J Biomed Mater Res 10: 311-323
- Koch RJ, Gorti GK. 2002. Tissue engineering with Chondrocytes. Facial Plast Surg 18:59-68.
- Kon E, Muraglia A, Corsi A, Bianco P, Marcacci M, Martin I. Boyde A, Rispantini I, Chistolini P, Rocca M, Giardino R, Cancedda R, Quarto R. 2000. Autologous bone marrow stromal cells loaded onto porous hydroxyapatite ceramic accelerate bone repair in critical-size defects of sheep long bones. J Biomed Mater Res 49: 328-337
- Li SH, De Groot K, Layrolle P. 2002. Bioceramic scaffold with controlled porous structure for bone tissue engineering. Key Engineering Materials 25: 218-220
- Lu L, Zhu X, Valenzuela RG, Currier BL, Yaszemski MJ. 2001. Biodegradable polymer scaffolds for cartilage tissue engineering. Clin Orthop 391S: 251-270.
- Martinetti R, Belpassi A, Nataloni A, Piconi C. 2001. Porous hydroxyapatite cell carrier for tissue engineering. In: Gianni S, Moroni A. (eds) Proceedings of the 13^{th} Int Symp On Ceramics in Medicine. Bologna, Italy Nov. 2000: 507-510. Trans Tech Publications, Switzerland
- Ohgushi H, Okumura M, Yoshikawa T, Inoue K, Senpuku N, Tamai.S. 1992. Bone formation process in porous calcium carbonate and hydroxyapatite. J Biomed Mater Res 26:885-895
- Takaoka K, Nakahara H, Yoshikikawa H, Masuhara K, Tsuda T, Ono K. 1988. Ectopic bone induction on and in porous hydroxyapatite combined with collagen and bone morphogenetic protein. Clin Orthop 234: 250-254
- Toquet J, Rohanizadeh R, Guicheux J, Couillaud S, Passuti N, Daculsi G, Heymann D. 1999. Osteogenic potential in vitro of human bone marrow cells cultured on macroporous biphasic calcium phosphate ceramic. J Biomed Mater Res 44: 98-108
- Weng Y, Cao Y, Silva CA, Vacanti MP, Vacanti CA. 2001. Tissue-engineered composites of bone and cartilage for mandible condylar reconstruction. J Oral Maxillof Surg 59:185-190
- Wilson C, De Bruijn J, Kruijt M, Van Gaalen S, Dhert W, Verbout A, Van Blitterswijk. 2001. Design and fabrication of porous hydroxyapatite scaffolds for bone tissue engineering using Rapid prototyping techniques. Orthopaedic Research Society 47^{th} Annual meeting, San Francisco USA.
- Yaszemski MJ, Payne RJ, Hayes WC, Langer R, Mikos AG. 1996. Evolution of bone transplantation: molecular, cellular and tissue strategies to engineer human bone. Biomaterials 17: 175-185

### Methods for obtaining inoculation cells are disclosed in e.g.:

- Friedstein AJ, Chailakhyan RK, Gerasimov UV. 1987. Bone marrow osteogenic stem cells: In vitro cultivation and transplantation in diffusion chambers, Cell Tissue Kinet 20: 263-272
- Goldstein A. 2001. Effect of Seeding Osteoprogenitor Cells as Dense Clusters on Cell Growth and Differentiation. Tissue Eng 7: 817-827
- Koller MR, Bradley MS, Palsson B. 1995. Growth factor consumption and production in perfusion cultures of human bone marrow correlate with specific cell production, Experimental haematology 23:1275-1283
- Owen ME. 1988. Marrow stromal stem cells. Cell Sci 10: 63-76
- Strehl R, Schumacher K, De Vries U, Minuth W. 2002. Proliferating cells versus Differentiated Cells in Tissue Engineering. Tissue Eng 8: 37-42

It shall be clear to the skilled man that the invention is not limited to the embodiment described herein and that many varations are possible within the scope of the appended claims.

## Claims

1. Bioreactor, comprising a rigid, portable outer housing enclosing a sterile medium chamber in which an inner housing is disposed, the outer housing being provided with a medium inlet port and a medium outlet port for sustaining a medium flow path through the medium chamber and with a medium chamber opening, openable and closable with an outer housing lid for allowing placement and removal of the inner housing, said inner housing being disposed in said flow path and having a medium permeable wall enclosing a growth chamber for growing cells on three-dimensional biomaterial scaffolds to a hybrid tissue engineered, a growth chamber opening, openable and closable with an inner housing lid for allowing placement of the scaffolds and removal of hybrid constructs grown therewith, and a sterile gripping surface, accessible when said medium chamber is open, for sterile removal of the inner housing from the outer housing.

2. Bioreactor according to claim 1, wherein the inner housing forms a rigid basket for the scaffolds.

3. Bioreactor according to claim 1 or 2, wherein said inner and outer housings are nested.

4. Bioreactor according to claim 3, wherein the mantle surface of the cylindrical inner housing is permeable for the medium and wherein the inner housing is arranged for radial flow in the flow path.

5. Bioreactor according to claim 3 wherein the end surfaces of the cylindrical inner housing are permeable for the medium and wherein the inner housing is arranged for axial flow in the flow path.

6. Bioreactor according to any of the preceding claims, wherein is the sterile gripping surface is part of a projection protruding from the inner housing.

7. Bioreactor according to any of claims 1-6, wherein the sterile gripping surface is located on the inner housing lid.

8. Bioreactor according to any of the preceding claims, wherein the inner housing is removably carried on the outer housing lid.

9. Bioreactor according to any of the preceding claims, wherein the medium permeable wall of the inner housing is interposed between an inner membrane and an outer membrane.

10. Bioreactor according to any of the preceding claims, wherein an inoculation chamber is arranged in the flow path.

11. Bioreactor according to any of the preceding claims, wherein a construct sampling chamber is provided in the flow path.

12. Bioreactor according to claim 11, wherein the sampling chamber is provided in the growth chamber as a removable unit.

13. Bioreactor according to any of the preceding claims, wherein the outer housing comprises an inoculation port.

14. Bioreactor according to any of the preceding claims, built-up from disposable plastics material.
